(19) Europäisches Patentamt
European Patent Office
Office européen des brevets

(11) Veröffentlichungsnummer : **0 145 840**
**B1**

(12) **EUROPÄISCHE PATENTSCHRIFT**

(45) Veröffentlichungstag der Patentschrift : **06.05.87**

(51) Int. Cl.[4] : **C 07 C127/19**

(21) Anmeldenummer : **84109848.6**

(22) Anmeldetag : **17.08.84**

(54) **Monoester von Celiprolol mit optisch aktiven disubstituierten Weinsäuren, deren Herstellung und Verwendung.**

(30) Priorität : **19.08.83 DE 3330005**

(43) Veröffentlichungstag der Anmeldung : **26.06.85 Patentblatt 85/26**

(45) Bekanntmachung des Hinweises auf die Patenterteilung : **06.05.87 Patentblatt 87/19**

(84) Benannte Vertragsstaaten : **AT BE CH DE FR GB IT LI LU NL SE**

(56) Entgegenhaltungen :
**AUSTRALIAN JOURNAL OF CHEMISTRY, Band 32, Nr. 1, Januar 1979, Seiten 65-70, Melbourne, AU; K.H. BELL: "Kinetic resolution in the reaction of (2R,3R)-2,3-diacetoxy- and (2R,3R)-2,3-dibenzoyloxy-succinic anhydrides with racemic alcohols"**
**CHEMICAL ABSTRACTS, Band 97, Nr. 9, 30. August 1982, Seite 566, no. 71606r, Columbus, Ohio, US; V.F. KOLOMIETS: "(S)-(-)-alpha-phenylethyl isocyanate and D-(+)-dibenzoyltartaric anhydride in the kinetic cleavage of alcohols and amines"**

(73) Patentinhaber : **CHEMIE LINZ AG**
**Postfach 296**
**A-4021 Linz (AT)**

(72) Erfinder : **Lindner, Wolfgang, Dr.**
**St. Veiter-Anger 22**
**A-8046 Graz (AT)**

EP 0 145 840 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Celiprololester von optisch aktiven disubstituierten Weinsäuren, deren Herstellung und Verwendung zur Herstellung optisch reiner Formen von Celiprolol.

Die DE-PS 2 458 624 beschreibt unter anderem das Racemat des 3-[3-Acetyl-4-(3-tert.butylamino-2-hydroxypropoxy-phenyl]-1,1-diäthylharnstoffs (Celiprolol) mit der nachfolgenden Formel

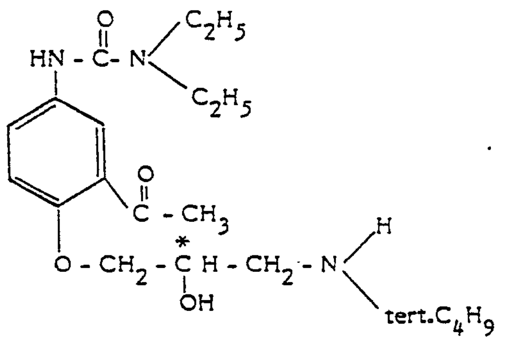

(III)

als Substanz mit beta$_1$-Rezeptoren blockierender Wirkung.

Beta-Blocker sind Substanzen mit wertvollen pharmakologischen Eigenschaften und haben große Bedeutung bei der Behandlung von Erkrankungen des Herzens und des Kreislaufs.

In der Struktur der Verbindung der Formel III ist das mit einem Stern gekennzeichnete Kohlenstoffatom in der basischen Seitenkette ein Chiralitätszentrum, sodaß Celiprolol in zwei optischen Antipoden vorliegen kann.

Für die Trennung solcher Racemate bestehen mehrere Möglichkeiten, wie sie bei anderen β-Blockern bereits Anwendung fanden. Eine Möglichkeit dazu besteht in der Salzbildung der racemischen β-Blocker mit optisch aktiven Säuren und Trennung des diastereomeren Salzpaares auf Grund unterschiedlicher Löslichkeitseigenschaften durch fraktionierte Kristallisation. Als solche Säuren, die zur Trennung von β-Blocker bereits herangezogen wurden, sind beispielsweise zu nennen Campher-10-sulfonsäure (P. Newman, Optical Resolution Procedures for Chemical Compounds, Vol. 1, Optical Resolution Information Center, New York, 10471). Obwohl sich aus den so erhaltenen optisch reinen Salzen leicht durch Alkalisieren die optisch reinen Alkanolamine gewinnen lassen, sind diese Verfahren meist doch nachteilig, da sie häufig mit mehreren Kristallisationsstufen verbunden sind, um eine ausreichende Trennung zu erzielen, sodaß relativ aufwendige Opterationen nötig sind, um allzu große Substanzverluste zu vermeiden.

Es ist ferner auch schon vorgeschlagen worden, racemische β-Blocker mit optisch reinen Reaktionspartnern umzusetzen und in diastereomere Reaktionsprodukte überzuführen, die dann auf Grund unterschiedlicher Eigenschaften in die optischen Antipoden aufgetrennt und zu den optisch reinen β-Blockern gespalten werden. Als solche optisch reine Reaktionspartner dienten z. B. im Falle des Propranolols oder Metoprolols tert. Butyloxycarbonyl-L-alaninanhydrid oder -L-Leucinanhydrid (J. Hermansson und C. von Bahr, J. Chromatogr. 227 (1982) 113), N-Trifluoracetyl-S(-)-prolylchlorid (S. Caccia et al, J. Chromatogr. Science (1978) 543) oder Aralkylisocyanate (W. Dieterle und W. Faigle, J. Chromatogr. 259 (1983) 311). Alle diese Reagenzien haben den Nachteil der schweren Zugänglichkeit und führen gewöhnlich durch Reaktion mit der Aminfunktion des Alkanolamins zu Produkten mit Amidstruktur. Abgesehen davon, daß sich solche Amide nur unter relativ scharfen Reaktionsbedingungen spalten lassen, was die Gefahr der Racemisierung mit sich bringt, ist auch die Stelle der Derivatisierung vom Chiralitätszentrum des Alkanolamins relativ weit entfernt, sodaß geringere Unterschiede in den chemischen und physikalischen Eigenschaften der Diastereomeren als im Fall einer O-Derivatisierung die Folge sind.

Der vorliegenden Erfindung liegt nun die Aufgabe zugrunde, einen Weg zur optischen Trennung von Celiprolol zu finden, der einfache Durchführbarkeit mit einem guten Trenneffekt verbindet und ohne langwierige Operationen zu Produkten mit guter optischer Reinheit führt.

Diese Aufgabe kann gelöst werden, wenn zur Trennung die überraschenderweise sehr stark unterschiedlichen chemischen und physikalischen Eigenschaften der Monoester von Celiprolol mit bestimmten optisch aktiven disubstituierten Weinsäuren herangezogen werden.

Gegenstand der vorliegenden Erfindung sind die Monoester von Celiprolol mit optisch aktiven disubstituierten Weinsäuren der Formel

(Siehe Formel Seite 3 f.)

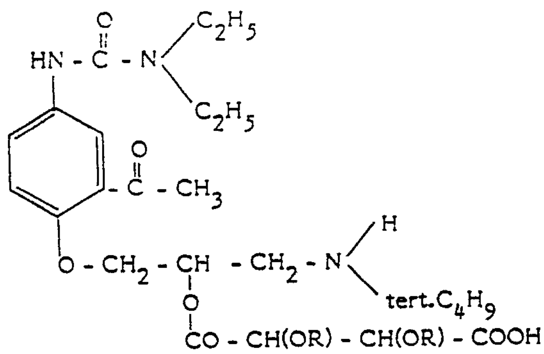

(I)

in der R einen Alkyl-, Aralkyl-, gegebenenfalls substituierten Alkanoyl-, gegebenenfalls substituierten Aroyl-, substituierten Vinyl- oder einen N-Arylcarbamoylrest bedeutet, sowie deren Salze mit Säuren oder Basen.

Diastereomerengemische der Monoester der Formel I sind auf Grund ihrer stark unterschiedlichen chemischen und physikalischen Eigenschaften auf einfache Weise in die optisch reinen Verbindungen aufzutrennen, wobei ohne langwierige Reinigungsoperationen gleich eine hohe optische Reinheit erzielbar ist. Besonders bewährt haben sich dabei als Dialkylweinsäuren solche, in denen der Alkylrest 1-5 Kohlenstoffatome, vorzugsweise 1-4 Kohlenstoffatome besitzt, wobei Methyl, Äthyl und tert.Butyl wiederum besonders bevorzugt sind. Als Diaralkylweinsäure ist bevorzugt die Dibenzylweinsäure einzusetzen. Der Alkanoylrest in Di-alkanoylweinsäuren kann beispielsweise durch einen Arylrest oder Halogen substituiert sein, wobei als Beispiel der Trichloracetylrest und der Phenylacetylrest hervorzuheben sind. Bevorzugt sind unsubstituierte Alkanoylreste mit 1-4 C-Atomen, wobei wiederum der Acetylrest besonders bevorzugt ist. Als Aroylrest in Diaroylweinsäuren kommen neben dem Benzoylrest der Toluoyl-, Nitrobenzoyl-, oder Dinitrobenzoylrest in Betracht, wobei der Benzoyl- und der Toluoylrest besonders bevorzugt sind. Bevorzugte Substituenten für den Vinylrest sind Halogen, wobei der β,β'-Dichlorvinylrest besonders hervorzuheben ist. Als N-Arylcarbamoylrest sind besonders der N-Phenylcarbamoylrest und der N-Naphthylcarbamoylrest zu nennen.

Ein weiterer Gegenstand der vorliegenden Erfindung sind die optisch reinen Weinsäuremonoester der Formel I, die aus den diastereomeren Weinsäureestern durch Trennung erhalten werden, sowie deren Salze.

Die Herstellung der erfindungsgemäßen diastereomeren Weinsäuremonoester der allgemeinen Formel I gelingt, indem man das racemische Gemisch des Celiprolols in der Schmelze oder in einem aprotischen Lösungsmittel mit einer disubstituierten (R,R)- oder (S,S)-Weinsäure der allgemeinen Formel

$$HOOC \cdot CH(OR){-}CH(OR){-}COOH \qquad (II),$$

in der R wie oben angegeben definiert ist oder mit deren Anhydrid umsetzt, wobei im Falle, daß die freie Säure zur Umsetzung verwendet wird, in Gegenwart eines Kondensationsmittels gearbeitet wird.

Als Kondensationsmittel können hierbei beispielsweise Carbodiimide wie Dicyclohexylcarbodiimid, Tosylchlorid, Trifluoressigsäureanhydrid, Sulfurylchlorid, ein Gemisch von Dimethylformamid und Thionylchlorid, Aluminiumoxid und Molekularsiebe genannt werden.

Da die Aminogruppe im Celiprolol sekundär ist, ist eine Reaktion der Weinsäure der Formel II oder von deren Anhydrid auch mit der Aminogruppe unter Amidbildung möglich. Überraschenderweise entstehen bei der erfindungsgemäßen Reaktion jedoch bevorzugt die diastereomeren O-Derivate und N-Derivate bzw. doppelt substituierte Derivate nur als Nebenprodukte. Durch Ausfällen können die O-Derivate von den unerwünschten N-Derivaten abgetrennt werden.

Zweckmäßigerweise wird man jedoch die Aminfunktion während der Umsetzung schützen, wozu jede für Aminogruppen übliche Schutzgruppe, die durch Verseifung oder Hydrierung abgespalten werden kann, in Betracht kommt. Besonders bevorzugt ist es jedoch, die Aminogruppe durch Salzbildung mit einer starken anorganischen oder organischen Säure zu schützen, die mit der Amionogruppe eine Ionenpaarbindung eingeht, die unter den Reaktionsbedingungen nur schwach dissoziiert. Diese Säure kann dann nach erfolgter Umsetzung durch Behandlung der resultierenden Ester mit schwachen Basen, beispielsweise wäßrigen $NaHCO_3$-Lösungen oder Aminen in Freiheit gesetzt werden. Es ist auch möglich, die Säure erst nach der Auftrennung der Weinsäuremonoester der Formel I in die optisch reinen Ester abzutrennen.

Als organische Säuren, die sich für diese Schutzfunktion sehr gut eignen, können Sulfonsäuren und Halogencarbonsäuren genannt werden, als anorganische Säuren kommen vor allem Mineralsäuren in Frage. Vorzugsweise werden Toluolsulfonsäure, Trichloressigsäure, Trifluoressigsäure oder Salzsäure eingesetzt.

Als bevorzugte Ausführungsform des erfindungsgemäßen Verfahrens ist die Umsetzung des Anhydrids der Weinsäure der Formel II mit Celiprolol in einem aprotischen Lösungsmittel bei Temperaturen von 5 °C bis 150 °C zu nennen. Zweckmäßigerweise liegt die Temperatur, die von dem verwendeten

Lösungsmittel sowie von der verwendeten Weinsäure abhängt im Bereich von Raumtemperatur bis 90 °C und bevorzugt zwischen 40 °C und 90 °C. Die Anhydride der Säuren der Formel II werden dabei äquimolar oder im Überschuß eingesetzt. Die Reaktionszeiten betragen in der Regel zwischen 2 und 36 Stunden.

Als aprotische Lösungsmittel sind bevorzugt Dichlormethan, 1,2-Dichloräthan, Aceton, Acetonitril, Toluol, Dimethylformamid, Dimethylsulfoxyd, Tetrahydrofuran, Dioxan, Äthylenglykoldimethyläther oder N-Methylpyrrolidon-2 zu nennen. Die Lösungsmittel werden in wasserfreiem Zustand eingesetzt. Wasser, das durch Reagentien, z. B. durch kristallwasserhältige p-Toluolsulfonsäure in die Reaktion eingeschleppt wird, wird zweckmäßig vor der Umsetzung durch Abdestillieren entfernt.

Die so gebildeten Gemische der diastereomeren Weinsäuremonoester der Formel I können aus wäßrigem Medium, bevorzugt im pH-Bereich von 0 bis 9 kristallisiert werden, nachdem das Lösungsmittel vorzugsweise durch Abdampfen entfernt worden ist. Es ist aber auch möglich, diese durch Extrahieren mit organischen Lösungsmitteln, bevorzugt Dichlormethan oder Dichloräthan, abzutrennen und das Lösungsmittel dann abzudestillieren.

Wird die Aminfunktion des Celiprolols durch Säuren geschützt, wird die Säure zweckmäßig durch Zufügen einer schwachen wäßrigen Base aus dem trockenen Reaktionsgemisch entfernt. Man kann sie aber auch durch Behandeln der Lösungen der Weinsäureester mit wäßrigen Alkalien eliminieren.

Die so erhaltenen diastereomeren ester der Formel I liegen auf Grund ihrer sauren und basischen Funktionen zwitterionisch bzw. als inneres Salz vor. Sie können aber auch durch Zusatz von Säuren in Säureadditionssalze und von Basen in Salze der Carbonsäurefunktion übergeführt werden.

Aus dem erhaltenen diastereoisomeren Estergemisch der Formel I können die optisch reinen Ester der Formel I durch Trennung auf Grund der unterschiedlichen physikalischen und chemischen Eigenschaften erhalten werden, wobei eine selektive Kristallisation oder Extraktion sowie eine chromatographische Trennung besonders geeignet sind.

Als Lösungsmittel, aus denen eine fraktionierte Kristallisation möglich ist, kommen Chlorkohlenwassersoffe, Ketone, wie Aceton, Äther, Ester, sekundäre und tertiäre Alkohole wie Isopropanol, Wasser oder wäßrige Pufferlösungen in Betracht. Meist gelangt man nach bereits 1 bis 4 Kristallisationsschritten zu den optisch reinen Weinsäureestern der Formel I.

Bevorzugt wird jedoch zur Trennung eine Extraktion oder eine chromatographische Trennung angewendet. Für die Extraktion werden bevorzugt Lösungsmittel gewählt, in denen die Ester der Formel I nicht oder nur langsam solvolysierbar sind. Als solche sind Chlorkohlenwasserstoffe, Ketone, Äther, Ester, sekundäre und tertiäre Alkohole, Wasser oder wäßrige Pufferlösungen zu nennen, wobei Aceton, Isopropanol, Dichlormethan, Dichloräthan, Chloroform, Essigester, Cyclohexan-Toluol, Tetrahydrofuran, Dioxan, Äther, Alkali- oder Ammoniumphosphatpufferlösungen bevorzugt sind.

Als chromatographische Trennverfahren sind sowohl die Dünnschichtchromatographie, die Säulenchromatographie als auch die Hochdruckflüssigkeitschromatographie geeignet. Bevorzugt sind die Adsorptionschromatographie an Kieselgelsäulen oder Aluminiumoxidsäulen mit Aceton, Isopropanol, Dichlormethan, Cyclohexan oder Toluol, vorzugsweise Aceton/Isopropanol als Laufmittel und die Hochdruckflüssigkeitschromatographie mit Reversed Phase-System, z. B. mit RP 18 (Reversed Phase-Octadecyl) als stationäre Phase. Als mobile Phase können im letzteren Fall saure Puffer mit Methanol oder Acetonitril gemischt dienen.

In der Flüssig-Chromatographie (LC oder HPLC) erreicht man Stofftrennungen auf Grund unterschiedlicher chemisch-physikalischer Wechselwirkungen innerhalb eines 3-Compartment Systems, den zu trennenden Substanzen, der stationären Phase und der mobilen Phase. In einem Reversed-Phase-System beruht die Auftrennung eines Gemisches z. B. bevorzugt auf Grund unterschiedlicher Lipophilie der Substanzen in der mobilen und stationären Phase, was auch durch unterschiedliche Verteilungskoeffizienten zu beschreiben ist. Unter konstanten isokratischen und isothermen Bedingungen (definierte stationäre Phase, z. B. RP 18, und definierte mobile Phase, z. B. bestimmtes Methanol-Puffer Gemisch) wird das Retentionszeitverhalten einer Substanz über ihre Gesamtretentionszeit beschrieben. Diese ist von diversen Säulenparametern und der Fließgeschwindigkeit der mobilen Phase abhängig. Um eine weitgehend unabhängige Beschreibung des Substanzverhaltens in einem Chromatographiesystem zu erhalten, formulierte man den Kapazitätsfaktor $k_i'$

$$k_i' = t_{ri} - t_0/t_0$$

i = Substanz i ; $t_r$ = Gesamtretentionszeit der Substanz i ;

$t_0$ = Totzeit einer nicht retardierten Substanz im gleichen chromatogr. System.

Um das Verhalten zweier Substanzen in ein und demselben Chromatographie-System auf einfache Weise zu beschrieben, definierte man die « Relative Retention », oder auch « Selektivitätsfaktor alpha », der somit die Auftrennbarkeit eines Substanzgemisches i-j angibt

$$alpha_{i,j} = k_j'/k_i'$$

(Substanz j wird länger retardiert als Substanz i.)

Hohe alpha-Werte bedeuten somit eine sehr selektive Auftrennung eines bestimmten Substanzgemi-

sches, welches in den nachfolgenden Beispielen jeweils ein diastereomeres Antipodenpaar darstellt. Die k' und alpha-Werte stellen somit chemisch-physikalische Größen dar, welche substanzspezifisch und substanzcharakteristisch in einem gegebenen Chromatographiesystem (stationäre Phase in Kombination mit einer bestimmten mobilen Phase) sind. (Literatur : Chromatogr. Trennmethoden, G. Schwendt, Georg Thieme Verlag Stuttgart, 1979).

Die auf diese Weise erhaltenen optisch reinen Weinsäuremonoester der Formel I sind in besonders günstiger Weise als Ausgangsmaterialien für die Herstellung der optisch reinen Formen des Celiprolols geeignet, da sie sich unter milden Bedingungen solvolytisch spalten lassen. Diese Spaltung gelingt meist im Temperaturbereich zwischen — 10° und 40 °C unter sauren oder basischen Bedingungen, z. B. mit 0,1 bis 1 n wäßriger Salzsäure oder 0,1 bis 1 n wäßriger NaOH oder durch Umesterung z. B. in Methanol.

Eine andere Möglichkeit zur Herstellung der optisch reinen Alkanolamine des Celiprolols besteht darin, daß man das Gemisch der diastereomeren Weinsäuremonoester der Formel I einer stereospezifischen, pH-kontrollierten Hydrolyse oder einer stereospezifischen enzymatischen Hydrolyse unterwirft. Der Weg über die Trennung der optisch reinen Weinsäuremonoester der Formel I ist jedoch bevorzugt.

Beispiel 1

18,15 g (0,05 Mol) (R,S)-Celiprolol basisch werden gemeinsam mit 9 g (0,055 Mol) Trichloressigsäure in 100 ml Dichlormethan bei Raumtemperatur gelöst, welche sodann mit $MgSO_4$ getrocknet wird. Zur vom Trocknungsmittel befreiten Lösung gibt man 17 g (0,079 Mol) (R,R)-O,O-Diacetylweinsäureanhydrid und rührt die Lösung bei Raumtemperatur. Nach 90 Minuten Reaktionszeit fügt man der organischen Lösung 50 ml gesättigte wäßrige $NaHCO_3$ Lösung zu und rührt kräftig. Nachdem man die Phasen getrennt hat, wiederholt man diesen Schritt nochmals. Die organische Phase wird sodann mit $MgSO_4$ getrocknet und hernach das Dichlormethan im Vakuum bei Raumtemperatur abrotavapiert. Als Rückstand erhält man eine amorphe Festsubstanz, ein Gemisch aus (R)- und (S)-Celiprolol-(R,R)-O,O-Diacetylweinsäure-mono-ester (Fraktion A und Fraktion B).

Ausbeute : 29 g Diastereomerengemisch (Ca. 90 % der Theorie) (Laut HPLC Analyse sind noch kleine Anteile Reagens vorhanden).

10 g Substanzgemisch der Diastereomeren Fraktion A und Fraktion B werden in 50 ml Aceton gelöst und auf eine präparative Kieselgelsäule, die mit 500 g Kieselgel Si60 (230-400 mesh) gefüllt ist, injiziert. Als Laufmittel dient ein Gemisch aus Aceton/Isopropanol (1/1). Fraktion B eluiert vor der Fraktion A und es gelingt auf diese Weise in guter Ausbeute Fraktion B mittels Fraktionsschneidens rein zu isolieren.

Ausbeute : 2 g (40 % der Theorie) Fraktion B ; linksdrehender Celiprolol-(R,R)-O,O-Diacetyl-weinsäure-mono-ester.

$$[\text{alpha}]_D^{20°C} = -41{,}3° \qquad (c = 1{,}0 \text{ Dichlormethan})$$

optische Reinheit größer als 98 %

2,2 g Mischfraktion sowie 2,4 g Fraktion A verunreinigt mit ca. 10 % Fraktion B ; 0,8 g (16 % der Theorie) Fraktion A ; rechtsdrehender Celiprolol-(R,R)-O,O-Diacetyl-weinsäure-mono-ester.

$$[\text{alpha}]_D^{20°C} = +54{,}5° \qquad (1{,}0 \text{ Dichlormethan})$$

optische Reinheit größer als 98 %.

Dünnschichtchromatographische Trennung des Diastereomerengemisches ; Kieselgelplatte Si60, Fa. Merck (Alufolie) ;

Laufmittel : Isopropanol/Aceton (1/1)

$R_f$ Werte = Fraktion B 0,46/Fraktion A 0,16

alpha = 2,87
(Laufstrecke 10 cm)
Spektroskopische Daten : $^1$H-NMR (DMSO-$d_G$) TMS als innerer Stand, delta (ppm)
Varian XL200 · CH der Weinsäure
Fraktion A delta = 4,93 und 4,86 ppm (d,l = 7,5 Hz)
Fraktion B delta = 5,50 und 5,20 ppm (d,l = 6,5 Hz).

Beispiel 2

18,4 g (50 mMol) (R,S)-Celiprolol basisch werden gemeinsam mit 9 g (55 mMol) Trichloressigsäure in 100 ml Dichlormethan bei Raumtemperatur gelöst. Diese Lösung wird sodann mit $MgSO_4$ getrocknet. Nach Entfernung des Trocknungsmittels gibt man 23,8 g (70 mMol) (R,R)-O,O-Dibenzoyl-weinsäureanhydrid zu und rührt die Lösung bei Raumtemperatur. Nach ca. 2 Stunden Reaktionszeit saugt man das überschüssige Reagens ab und schüttelt die organische Lösung zweimal mit je 50 ml gesättigter wäßriger $NaHCO_3$ Lösung und wäscht einmal mit 50 ml reinem $H_2O$. Die verbleibende organische Phase wird,

nachdem sie mit $MgSO_4$ getrocknet wurde, am Rotovapor bei Raumtemperatur bis zur Trockene eingeengt, wobei man einen festen amorphen Rückstand, bestehend aus (R)- bzw. (S)-Celiprolol-O,O-Dibenzoyl-weinsäure-mono-ester erhält. Ausbeute : 33 g Diastereomerengemisch (ca. 90 % der Theorie) der Fraktionen I und II. (Laut HPLC Analyse sind noch kleine Anteile Reagens vorhanden).

10 g Diastereomerengemisch aus Fraktion I und Fraktion II werdem in 50 ml Aceton gelöst und auf eine präparative Kieselgelsäule, die mit 500 g Kieselgel Si60 (230-400 mesh) gefüllt ist, aufgetragen und mit dem Laufmittel Aceton/Isopropanol (18/1) in die Fraktionen I und II aufgetrennt. Fraktion II eluiert vor der Fraktion I und es gelingt auf diese Weise, Fraktion II in guter Ausbeute mittels Fraktionsschneidens zu erhalten.

Ausbeute : 4,1 g (82 % der Theorie) Fraktion II linksdrehender Celiprolol-(R,R)-O,O-Dibenzoyl-weinsäure-mono-ester, welche mit ca. 10 % Fraktion I verunreinigt ist. Davon läßt sich einfach 3,5 g (70 % der Theorie) reine Fraktion II durch Wiederholung der Säulen-Trennung unter gleichen Bedingungen erhalten.

$$[alpha]_D^{20 °C} = -62{,}6°$$ (c = 1,0 Dichlormethan)

optische Reinheit größer als 98 % ;

$^1$H-NMR ($CDCl_3$) TMS als innerer Standard, delta (ppm) ; Varian XL200 ; CH der (R,R)-Weinsäure ; delta = 5,56 und 5,45 ppm (d. I. = 7 Hz).

Dünnschichtchromatographische Trennung des Diastereomerengemisches Kieselgelplatte (Alufolie) Si60, Fa. Merck ;

Laufmittel : Isopropanol/Aceton (18/1).

$R_f$ Werte = Fraktion II 0,50/Fraktion I 0,09

alpha = 5,55

(Laufstrecke 11 cm)

Mittels der Dünnschichtchromatographie kann somit die säulenchromatographische Fraktion gut und in einfacher Weise verfolgt und kontrolliert werden.

## Beispiel 3

9 g (25 mM) (R,S)-Celiprolol basisch werden gemeinsam mit 5 g (60 mM) Trichloressigsäure in 100 ml Dichlormethan bei Raumtemperatur gelöst. Diese Lösung wird sodann mit $MgSO_4$ getrocknet. Nach Entfernung des Trocknungsmittels gibt man 9 g (80 mM) (S,S)-O,O-Diacetyl-weinsäure-anhydrid zu und rührt die Lösung bei Raumtemperatur. Nach 90 Minuten Reaktionszeit wird die organische Lösung zweimal mit 25 ml wäßriger gesättigter $NaHCO_3$-Lösung ausgeschüttelt und nochmals mit $H_2O$ gewaschen. Nach dem Trocknen der organischen Phase mit $MgSO_4$ wird diese bis zur Trockene am Rotovapor eingeengt, wobei man ein amorphes Pulver bestehend aus (R)- und (S)-Celiprolol-(S,S)-O,O-Diacetyl-weinsäure-mono-ester (Fraktion a und Fraktion b) erhält. Ausbeute 13 g Diastereomerengemisch (ca. 85 % der Theorie). Dieses Diastereomerengemisch läßt sich auf gleiche Weise wie in Beispiel 1 beschrieben adsorptionschromatographisch in seine optischen Antipoden auftrennen, wobei es jedoch zu einer Umkehr der Peakreihenfolge kommt. Fraktion b, welche vor der Fraktion a eluiert wird, ist nun der rechtsdrehende Celiprolol-(S,S)-O,O-Diacetyl-weinsäure-mono-ester. Fraktion a ist das korrespondierende Isomere, nämlich der linksdrehende Celiprolol-(S,S)-O,O-Diacetyl-weinsäure-mono-ester.

Die Fraktion B von Beispiel 1 und die Fraktion b im gegenständlichen Beispiel verhalten sich enantiotrop (ebenso Fraktion A und a), das heißt, sie zeigen gleiche chromatographische Eigenschaften in nicht chiralen Chromatographiesystemen. Säulenchromatographische Trennung und Bedingungen wie Beispiel 1.

Ausbeute : 1,9 g (38 % der Theorie) Fraktion b rechtsdrehender Celiprolol-(S,S)-O,O-Diacetyl-weinsäure-mono-ester.

$$[alpha]_D^{21 °C} = 40{,}9°$$ (c = 1,0 Dichlormethan)

Dünnschichtchromatographische Trennung des Diastereomerengemisches : Kieselgelplatte (Alufolie) Si60, Fa. Merck ; Laufmittel : Isopropanol/Aceton (1/1) ; Laufstrecke 11 cm.

$R_f$ Werte = Fraktion b 0,46/Fraktion a 0,16 alpha = 2,87

## Beispiel 4

Analog zu Beispiel 1 wurde Celiprolol mit (R,R)-O,O-Diäthylweinsäure zu den entsprechenden (R,R)-O,O-Diäthylweinsäuremonoestern des Celiprolols umgesetzt.

Die Trennung erfolgte über Reversed Phase HPLC.

Chromatographisches Trennsystem.

Säule 250 × 4,6 mm i. D. gepackt mit Spherisorb RP 18 ; Mobile Phase : 0,1 M Phosphorsäure mit wäßriger konz. 5 µm $NH_3$ auf pH 3,6 eingestellt — MeOH/50-50.

$k_{R'} = 0{,}62$, $k_{S'} = 1{,}0$, alpha = 1,61.

Die (R,R)- bzw. gegebenenfalls (S,S)-O,O-Diacyl, Dialkyl sowie Dibenzylweinsäuren bzw. deren Anhydride, welche in den verschiedenen Beispielen Verwendung finden, wurden gemäß bekannter Literaturverfahren hergestellt.

Beispiel 5

Analog zu Beispiel 1 wurde (R,S)-Celiprolol basisch mit (R,R)-O,O-Di(β-Dichlorvinyl)-weinsäureanhydrid umgesetzt. Die Trennung erfolgte über Reversed Phase HPLC

Chromatographisches Trennsystem :

Säule wie in Beispiel 6 ; Mobile Phase : 0,1 M Phosphorsäure mit wäßriger konzentrierter $NH_3$ auf pH 3,6 eingestellt — MeOH/40-60.

$k_R' = 1{,}58$, $k_S' = 2{,}63$, alpha 1,66

Die verwendete (R,)-O,O-Di(β-Dichlorvinyl)-weinsäure wurde von Shan und Alimchandani, J. Indian Chem. Soc. 11, 548 (1934) als (R,R)-O,O-(β-Dichlorethyl)-weinsäure bezeichnet. Sie wurde analog von uns hergestellt, durch Extraktion mit Äther isoliert und aus $CH_2Cl_2/CHCl_3$ umkristallisiert.

Fp = 125 °C [alpha] $^{22°C}_{546}$ = — 49,1 (c = 1,0 Methanol)

Spektroskopische Untersuchungen zeigten, daß es sich um eine β-Dichlorvinyl-Verbindung handelt.

**Patentansprüche** (für die Vertragsstaaten : BE, CH, DE, FR, GB, IT, LI, LU, NL, SE)

1. Monoester von Celiprolol mit optisch aktiven disubstituierten Weinsäuren der Formel

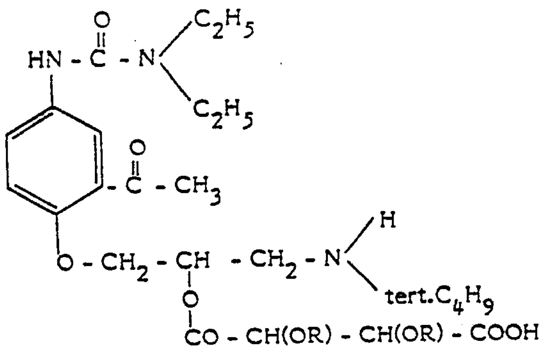

(I)

in der R einen Alkyl-, Aralkyl-, gegebenenfalls substituierten Alkanoyl-, gegebenenfalls substituierten Aroyl-, substituierten Vinyl- oder N-Arylcarbamoylrest bedeutet, sowie deren Salze mit Säuren oder Basen.

2. Monoester von Celiprolol gemäß Anspruch 1, dadurch gekennzeichnet, daß R einen Alkylrest mit 1-4 C-Atomen, den β,β'-Dichlorvinylrest, den Benzylrest, einen Alkanoylrest mit maximal 4 Kohlenstoffatomen, den Benzoyl- oder Toluoylrest, den N-Phenylcarbamoyl- oder den N-Naphthylcarbamoylrest bedeutet.

3. Monoester von Celiprolol gemäß den Ansprüchen 1 und 2, dadurch gekennzeichnet, daß sie in optisch reiner Form vorliegen.

4. Verfahren zur Herstellung von Monoestern von Celiprolol gemäß den Ansprüchen 1 und 2, dadurch gekennzeichnet, daß ein Gemisch der optischen Antipoden des Celiprolols in der Schmelze oder in einem aprotischen Lösungsmittel mit einer disubstituierten (R,R)- oder (S,S)-Weinsäure der allgemeinen Formel

$$HOOC—CH(OR)—CH(OR)—COOH \qquad (II)$$

in der R wie oben angegeben definiert ist oder mit deren Anhydrid umgesetzt wird, mit der Maßgabe, daß bei Umsetzung mit der freien Säure ein Kondensationsmittel zugegen ist, worauf das gebildete Antipodenpaargemisch der Monoester des Celiprolols isoliert und gewünschtenfalls in Salze übergeführt wird.

5. Verfahren nach Anspruch 4, dadurch gekennzeichnet, daß die sekundäre Aminogruppe des Celiprolols vor der Umsetzung geschützt und die Schutzgruppe nach erfolgtem Umsatz mit der disubstituierten Weinsäure der Formel II oder deren Anhydrid wieder abgespalten wird.

6. Verfahren nach Anspruch 5, dadurch gekennzeichnet, daß der Schutz der Aminogruppe durch

Umsetzung des Celiprolols mit einer anorganischen oder organischen Säure durchgeführt wird, die mit der Aminogruppe eine Ionenpaarbindung eingeht, die unter den Reaktionsbedingungen nur schwach dissoziiert und diese Schutzgruppe nach erfolgter Reaktion durch Alkalisieren wieder entfernt wird.

7. Verfahren nach Anspruch 6, dadurch gekennzeichnet, daß als Säuren Toluolsulfonsäure, Trichloressigsäure, Trifluoressigsäure oder Salzsäure eingesetzt werden.

8. Verfahren nach den Ansprüchen 4 bis 7, dadurch gekennzeichnet, daß die Veresterung in einem wasserfreien aprotischen Lösungsmittel bei Temperaturen zwischen Raumtemperatur und 90 °C durchgeführt wird.

9. Verfahren nach Anspruch 8, dadurch gekennzeichnet, daß als aprotisches Lösungsmittel Dichlormethan, 1,2-Dichloräthan, Aceton, Acetonitril, Toluol, Dimethylsulfoxyd, Tetrahydrofuran, Dioxan, Äthylenglykoldimethyläther oder N-Methylpyrrolidon-2 verwendet werden.

10. Verfahren zur Herstellung von optisch reinen Monoestern des Celiprolols gemäß Anspruch 3, dadurch gekennzeichnet, daß gemäß den Ansprüchen 4 bis 9 hergestellte Antipodenpaare der Monoester der Formel I durch selektive Kristallisation, Extraktion mit einem organischen Lösungsmittel, in denen die Ester der Formel I nicht oder nur langsam solvolysierbar sind, oder auf chromatographischem Weg getrennt werden.

11. Verfahren nach Anspruch 10, dadurch gekennzeichnet, daß die Trennung durch Adsorptionschromatographie an Kieselgelsäulen mit Aceton-Isopropanol als Laufmittel durchgeführt wird.

12. Verfahren nach Anspruch 10, dadurch gekennzeichnet, daß die Trennung durch Hochdruck-Flüssig-Chromatographie mit Reversed-Phase-System durchfeführt wird.

13. Verfahren nach Anspruch 12, dadurch gekennzeichnet, daß ein Methanol-Puffergemisch als mobile Phase dient.

14. Verwendung der Monoester des Celiprolols gemäß den Ansprüchen 1 bis 3 zur Herstellung optisch reiner Formen des Celiprolols durch hydrolytische Spaltung.

15. Verwendung nach Anspruch 14, dadurch gekennzeichnet, daß die optisch reinen Ester gemäß Anspruch 3 der Trennung unterworfen werden.

**Patentansprüche** (für den Vertragsstaat AT)

1. Verfahren zur Herstellung von Monoestern von Celiprolol mit optisch aktiven disubstituierten Weinsäuren der Formel

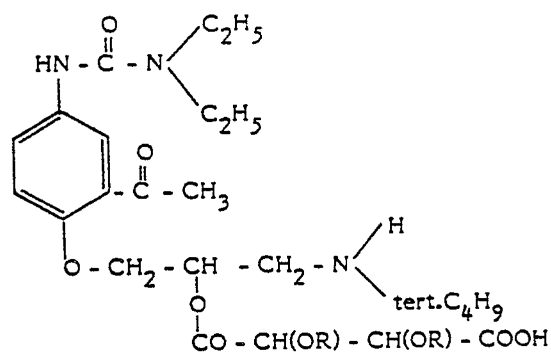

(I)

in der R einen Alkyl-, Aralkyl-, gegebenenfalls substituierten Alkanoyl, -gegebenenfalls substituierten Aroyl-, substituierten Vinyl- oder N-Aryl-carbamoylrest bedeutet, sowie deren Salze mit Säuren oder Basen, dadurch gekennzeichnet, daß ein Gemisch der optischen Antipoden des Celiprolols in der Schmelze oder in einem aprotischen Lösungsmittel mit einer disubstituierten (R,R)- oder (S,S)-Weinsäure der allgemeinen Formel

HOOC · CH(OR)—CH(OR)—COOH (II),

in der R wie oben angegeben definiert ist oder mit deren Anhydrid umgesetzt wird, mit der Maßangabe, daß bei Umsetzung mit der freien Säure ein Kondensationsmittel zugegen ist, worauf das gebildete Antipodenpaargemisch der Monoester des Celiprolols isoliert und gewünschtenfalls in Salze übergeführt wird.

2. Verfahren nach Anspruch 1, dadurch gekennzeichnet, daß man als disubstituierte Weinsäure der Formel II eine Verbindung einsetzt, in der R einen Alkylrest mit 1-4 C-Atomen, den β,β'-Dichlorvinylrest, den Benzylrest, einen Alkanoylrest mit maximal 4 Kohlenstoffatomen, den Benzoyl- oder Toluoylrest, den N-Phenylcarbamoyl- oder den N-Naphtylcarbamoylrest bedeutet.

3. Verfahren nach Anspruch 1 oder 2, dadurch gekennzeichnet, daß die sekundäre Aminogruppe des Celiprolols vor der Umsetzung geschützt und die Schutzgruppe nach erfolgtem Umsatz mit der disubstituierten Weinsäure der Formel II oder deren Anhydrid wieder abgespalten wird.

4. Verfahren nach Anspruch 3, dadurch gekennzeichnet, daß der Schutz der Aminogruppe durch Umsetzung des Celiprolols mit einer anorganischen oder organischen Säure durchgeführt wird, die mit der Aminogruppe eine Ionenpaarbindung eingeht, die unter den Reaktionsbedingungen nur schwach dissoziiert und diese Schutzgruppe nach erfolgter Reaktion durch Alkalisieren wieder entfernt wird.

5. Verfahren nach Anspruch 4, dadurch gekennzeichnet, daß als Säuren Toluolsulfonsäure, Trichloressigsäure, Trifluoressigsäure oder Salzsäure eingesetzt werden.

6. Verfahren nach den Ansprüchen 1 bis 5, dadurch gekennzeichnet, daß die Veresterung in einem wasserfreien aprotischen Lösungsmittel bei Temperaturen zwischen Raumtemperatur und 90 °C durchgeführt wird.

7. Verfahren nach Anspruch 6, dadurch gekennzeichnet, daß als aprotisches Lösungsmittel Dichlormethan, 1,2-Dichloräthan, Aceton, Acetonitril, Toluol, Dimethylsulfoxyd, Tetrahydrofuran, Dioxan, Äthylenglykoldimethyläther oder N-Methylpyrrolidon-2 verwendet werden.

8. Verfahren zur Herstellung von optisch reinen Monoestern des Celiprolols, dadurch gekennzeichnet, daß gemäß den Ansprüchen 1 bis 7 hergestellte Antipodenpaare der Monoester der Formel I durch selektive Kristallisation, Extraktion mit einem organischen Lösungsmittel, in denen die Ester der Formel I nicht oder nur langsam solvolysierbar sind, oder auf chromatographischem Weg getrennt werden.

9. Verfahren nach Anspruch 8, dadurch gekennzeichnet, daß die Trennung durch Adsorptionschromatographie an Kieselgelsäulen mit Aceton-Isopropanol als Laufmittel durchgeführt wird.

10. Verfahren nach Anspruch 8, dadurch gekennzeichnet, daß die Trennung durch Hochdruck-Flüssig-Chromatographie mit Reversed-System durchgeführt wird.

11. Verfahren nach Anspruch 10, dadurch gekennzeichnet, daß ein Methanol-Puffergemisch als mobile Phase dient.

12. Verwendung der Monoester des Celiprolols gemäß den Ansprüchen 1 bis 11 zur Herstellung optisch reiner Formen des Celiprolols durch hydrolytische Spaltung.

13. Verwendung nach Anspruch 12, dadurch gekennzeichnet, daß die optisch reinen Ester gemäß Anspruch 8 der Trennung unterworfen werden.

**Claims** (for the Contracting States : BE, CH, DE, FR, GB, IT, LI, LU, NL, SE)

1. Monoesters of celiprolol with optically active disubstituted tartaric acids, of the formula

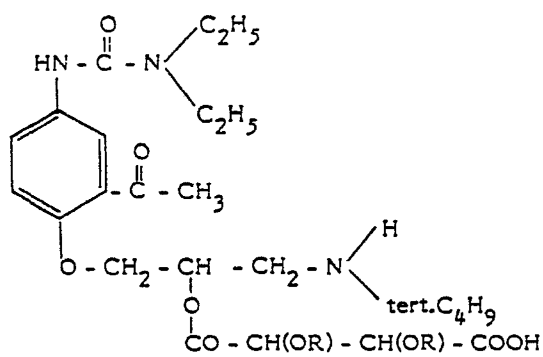

(I)

in which R denotes an alkyl, aralkyl, optionally substituted alkanoyl, optionally substituted aroyl, substituted vinyl or N-arylcarbamoyl radical, and their salts with acids or bases.

2. Monoesters of celiprolol according to Claim 1, characterized in that R denotes an alkyl radical having 1-4 C atoms, the β,β'-dichlorovinyl radical, the benzyl radical, an alkanoyl radical having a maximum of 4 carbon atoms, the benzoyl or toluoyl radical, or the N-phenylcarbamoyl or N-naphthylcarbamoyl radical.

3. Monoesters of celiprolol according to Claims 1 and 2, characterized in that they are in optically pure form.

4. Process for the preparation of monoesters of celiprolol according to Claims 1 and 2, characterized in that a mixture of the optical antipodes of celiprolol is reacted in the melt or in an aprotic solvent with a disubstituted (R,R)- or (S,S)-tartaric acid of the general formula

$$HOOC—CH(OR)—CH(OR)—COOH \quad (II)$$

in which R is defined as indicated above, or with its anhydride, with the proviso that for reaction with the free acid a condensing agent is present, and then the mixture of pairs of antipodes of celiprolol monoesters which has formed is isolated and, if desired, converted into salts.

5. Process according to Claim 4, characterized in that the secondary amino group of celiprolol is protected before the reaction and, after the reaction with the disubstituted tartaric acid of the formula II or its anhydride has taken place, the protective group is eliminated again.

6. Process according to Claim 5, characterized in that the amino group is protected by reacting celiprolol with an inorganic or organic acid which undergoes with the amino group ion-pair bonding which, under the reaction conditions, dissociates only slightly, and, after the reaction has taken place, this protective group is removed again by alkali treatment.

7. Process according to Claim 6, characterized in that the acids used are toluenesulphonic acid, trichloroacetic acid, trifluoroacetic acid or hydrochloric acid.

8. Process according to Claims 4 to 7, characterized in that the esterification is carried out in an anhydrous aprotic solvent at temperatures between room temperature and 90 °C.

9. Process according to Claim 8, characterized in that the aprotic solvent used is dichloromethane, 1,2-dichloroethane, acetone, acetonitrile, toluene, dimethyl sulphoxide, tetrahydrofuran, dioxane, ethylene glycol dimethyl ether or N-methyl-2-pyrrolidone.

10. Process for the preparation of optically pure monoesters of celiprolol according to Claim 3, characterized in that pairs of antipodes of the monoesters of the formula I prepared to Claims 4 to 9 are separated by selective crystallization, by extraction with an organic solvent in which the esters of the formula I can be solvolysed either not al all or only slowly, or by chromatographic means.

11. Process according to Claim 10, characterized in that the separation is carried out by adsorption chromatography on silica gel columns using acetone/isopropanol as mobile phase.

12. Process according to Claim 10, characterized in that the separation is carried out by high-pressure liquid chromatography with a reversed phase system.

13. Process according to Claim 12, characterized in that a methanol/buffer mixture is used as mobile phase.

14. Use of the monoesters of celiprolol according to Claims 1 to 3 for the preparatrion of optically pure forms of celiprolol by hydrolytic cleavage.

15. Use according to Claim 14, characterized in that the optically pure esters according to Claim 3 are subjected to separation.

**Claims** (for the Contracting State AT)

1. Process for the preparation of monoesters of celiprolol with optically active disubstituted tartaric acids, of the formula

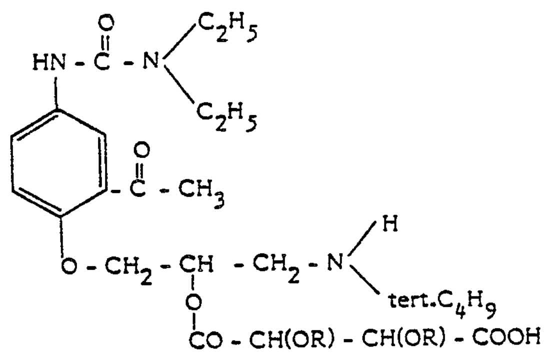

(I)

in which R denotes an alkyl, aralkyl, optionally substituted alkanoyl, optionally substituted aroyl, substituted vinyl or N-arylcarbamoyl radical, and their salts with acids or bases, characterized in that a mixture of the optical antipodes of celiprolol is reacted in the melt or in an aprotic solvent with a disubstituted (R,R)- or (S,S)-tartaric acid of the general formula

$$HOOC \cdot CH(OR)—CH(OR)—COOH \qquad (II)$$

in which R is defined as indicated above, or with its anhydride, with the proviso that for reaction with the free acid a condensing agent is present, and then the mixture of pairs of antipodes of celiprolol monoesters which has formed is isolated and, if desired, converted into salts.

2. Process according to Claim 1, characterized in that the disubstituted tartaric acid of the formula II which is used is a compound in which R denotes an alkyl radical having 1-4 C atoms, the β,β'-dichlorovinyl radical, the benzyl radical, an alkanoyl radical having a maximum of 4 carbon atoms, the benzoyl or toluoyl radical, or the N-phenylcarbamoyl or N-naphthylcarbamoyl radical.

3. Process according to Claim 1 or 2, characterized in that the secondary amino group of celiprolol is protected before the reaction and, after the reaction with the disubstituted tartaric acid of the formula II or its anhydride has taken place, the protective group is eliminated again.

4. Process according to Claim 3, characterized in that the amino group is protected by reacting celiprolol with an inorganic or organic acid which undergoes with the amino group ion-pair bonding which, under the reaction conditions, dissociates only slightly, and, after the reaction has taken place, this protective group is removed again by alkali treatment.

5. Process according to Claim 4, characterized in that the acids used are toluenesulphonic acid, trichloroacetic acid, trifluoroacetic acid or hydrochloric acid.

6. Process according to Claims 1 to 5, characterized in that the esterification is carried out in an anhydrous aprotic solvent at temperatures between room temperature and 90 °C.

7. Process according to Claim 6, characterized in that the aprotic solvent used is dichloromethane, 1,2-dichloroethane, acetone, acetonitrile, toluene, dimethyl sulphoxide, tetrahydrofuran, dioxane, ethylene glycol dimethyl ether or N-methyl-2-pyrrolidone.

8. Process for the preparation of optically pure monoesters of celiprolol, characterized in that pairs of antipodes of the monoesters of the formula I prepared according to Claims 1 to 7 are separated by selective crystallization, by extraction with an organic solvent in which the esters of the formula I can be solvolysed either not at all or only slowly, or by chromatographic means.

9. Process according to Claim 8, characterized in that the separation is carried out by adsorption chromatography on silica gel columns using acetone/isopropanol as mobile phase.

10. Process according to Claim 8, characterized in that the separation is carried out by high-pressure liquid chromatography with a reversed phase system.

11. Process according to Claim 10, characterized in that a methanol/buffer mixture is used as mobile phase.

12. Use of the monoesters of celiprolol according to Claims 1 to 11 for the preparation of optically pure forms of celiprolol by hydrolytic cleavage.

13. Use according to Claim 12, characterized in that the optically pure esters according to Claim 8 are subjected to separation.

**Revendications** (pour les Etats contractants : BE, CH, DE, FR, GB, IT, LI, LU, NL, SE)

1. Monoesters de céliprolol avec des acides tartriques disubstitués optiquement actifs, de formule :

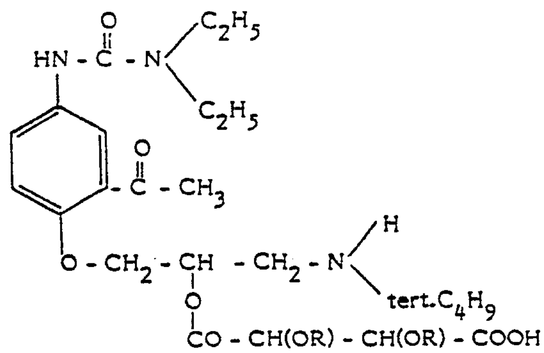

(I)

dans laquelle R représente un radical alcoyle, arylalcoyle, alcanoyle éventuellement substitué, aroyle éventuellement substitué, vinyle substitué ou N-arylcarbamoyle, ainsi que leurs sels avec des acides ou des bases.

2. Monoesters de céliprolol suivant la revendication 1, caractérisés en ce que R représente un radical alcoyle ayant 1-4 atomes de carbone, le radical β,β'-dichlorovinyle, le radical benzyle, un radical alcanoyle ayant au maximum 4 atomes de carbone, le radical benzoyle ou toluoyle, le radical N-phénylcarbamoyle ou le radical N-naphtylcarbamoyle.

3. Monoesters de céliprolol suivant les revendications 1 et 2, caractérisés en ce qu'ils se présentent sous forme optiquement pure.

4. Procédé pour la préparation de monoesters de céliprolol suivant les revendications 1 et 2, caractérisé en ce que l'on fait réagir un mélange des antipodes optiques du céliprolol dans la masse fondue ou dans un solvant aprotique avec un acide (R,R)- ou (S,S)-tartrique disubstitué de formule générale :

$$HOOC—CH(OR)—CH(OR)—COOH \quad (II)$$

dans laquelle R est défini comme mentionné ci-dessus ou avec son anhydride en proportion donnée, à condition qu'un agent de condensation soit présent dans le cas d'une réaction avec l'acide libre, après quoi on isole le mélange de la paire d'antipodes du monoester de céliprolol formé et on le transforme au cas où on le désire en sels.

5. Procédé suivant la revendication 4, caractérisé en ce que l'on protège le groupe amino secondaire du céliprolol avant la réaction et que l'on clive de nouveau le groupe protecteur une fois que la réaction avec l'acide tartrique disubstitué de formule (II) ou de son anhydride est terminée.

6. Procédé suivant la revendication 5, caractérisé en ce que l'on effectue la protection du groupe amino par réaction du céliprolol avec un acide inorganique ou organique entrant avec le groupe amino en

une liaison d'appariement ionique qui n'est que faiblement dissociée dans les conditions de réaction et on élimine de nouveau ce groupe protecteur par alcalinisation une fois la réaction terminée.

7. Procédé suivant la revendication 6, caractérisé en ce que l'on utilise comme acides, l'acide toluène-sulfonique, l'acide trichloracétique, l'acide trifluoracétique ou l'acide chlorhydrique.

8. Procédé suivant les revendications 4 à 7, caractérisé en ce que l'on effectue l'estérification dans un solvant aprotique anhydre à des températures comprises entre la température ambiante et 90 °C.

9. Procédé suivant la revendication 8, caractérisé en ce que l'on utilise comme solvant aprotique, le dichlorométhane, le 1,2-dichloréthane, l'acétone, l'acétonitrile, le toluène, le diméthylsulfoxyde, le tétrahydrofuranne, le dioxanne, l'éthylèneglycoldiméthyléther ou la N-méthylpyrrolidone-2.

10. Procédé pour la préparation de monoesters du céliprolol optiquement purs suivant la revendication 3, caractérisé en ce que l'on sépare les paires d'antipodes des monoesters de formule I préparées suivant les revendications 4 à 9 par cristallisation sélective, extraction avec un solvant organique dans lequel les esters de formule I ne sont pas ou seulement lentement solvolysables, ou par voie chromatographique.

11. Procédé suivant la revendication 10, caractérisé en ce que l'on effectue la séparation par chromatographie par adsorption sur des colonnes de gel de silice avec un mélange d'acétone/isopropanol en tant qu'éluant.

12. Procédé suivant la revendication 10, caractérisé en ce que l'on effectue la séparation par chromatographie liquide sous haute pression avec un système à phases inversées.

13. Procédé suivant la revendication 12, caractérisé en ce qu'un mélange de méthanol/tampon sert de phase mobile.

14. Application des monoesters du céliprolol suivant les revendications 1 à 3 à la préparation de formes du céliprolol optiquement pures par clivage hydrolytique.

15. Application suivant la revendication 14, caractérisée en ce que les esters optiquement purs suivant la revendication 3 sont soumis à la séparation.

**Revendications** (pour l'Etat contractant AT)

1. Procédé pour la préparation de monoesters de céliprolol avec des acides tartriques disubstitués optiquement actifs de formule :

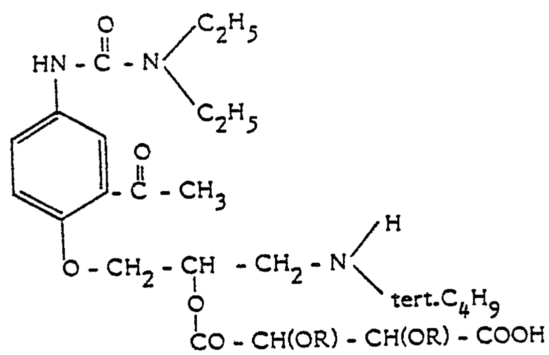

(I)

dans laquelle R représente un radical alcoyle, arylalcoyle, alcanoyle éventuellement substitué, aroyle éventuellement substitué, vinyle substitué ou N-arylcarbamoyle, ainsi que leurs sels avec des acides ou des bases, caractérisé en ce que l'on fait réagir un mélange des antipodes optiques du céliprolol dans la masse fondue ou dans un solvant aprotique avec un acide (R,R)- ou (S,S)-tartrique disubstitué de formule générale :

$$HOOC—CH(OR)—CH(OR)—COOH \qquad (II)$$

dans laquelle R est défini comme mentionné ci-dessus ou avec son anhydride en proportion donnée, à condition qu'un agent de condensation soit présent dans le cas d'une réaction avec l'acide libre, après quoi on isole le mélange de la paire d'antipodes du monoester de céliprolol formé et on le transforme au cas où on le désire en sels.

2. Procédé suivant la revendication 1, caractérisé en ce que l'on utilise comme acide tartrique disubstitué de formule II un composé dans lequel R représente un radical alcoyle ayant 1-4 atomes de carbone, le radical β,β'-dichlorovinyle, le radical benzyle, un radical alcanoyle ayant au maximum 4 atomes de carbone, le radical benzoyle ou toluoyle, le radical N-phénylcarbamoyle ou le radical N-naphtylcarbamoyle.

3. Procédé suivant la revendication 1 ou 2, caractérisé en ce que l'on protège le groupe amino secondaire du céliprolol avant la réaction et que l'on clive de nouveau le groupe protecteur une fois que la réaction avec l'acide tartrique disubstitué de formule II ou de son anhydride est terminée.

4. Procédé suivant la revendication 3, caractérisé en ce que l'on effectue la protection du groupe amino par réaction du céliprolol avec un acide inorganique ou organique entrant avec le groupe amino en une liaison d'appariement ionique qui n'est que faiblement dissociée dans les conditions de réaction et on élimine de nouveau ce groupe protecteur par alcalinisation une fois la réaction terminée.

5. Procédé suivant la revendication 4, caractérisé en ce que l'on utilise comme acides, l'acide toluènesulfonique, l'acide trichloracétique, l'acide trifluoracétique ou l'acide chlorhydrique.

6. Procédé suivant les revendications 1 à 5, caractérisé en ce que l'on effectue l'estérification dans un solvant aprotique anhydre à des températures comprises entre la température ambiante et 90°C.

7. Procédé suivant la revendication 6, caractérisé en ce que l'on utilise comme solvant aprotique, le dichlorométhane, le 1,2-dichloréthane, l'acétone, l'acétonitrile, le toluène, le diméthylsulfoxyde, le tétrahydrofuranne, le dioxanne, l'éthylèneglycoldiméthyléther ou la N-méthylpyrrolidone-2.

8. Procédé pour la préparation de monoesters du céliprolol optiquement purs, caractérisé en ce que l'on sépare les paires d'antipodes des monoesters de formule I préparées suivant les revendications 1 à 7, par cristallisation sélective, extraction avec un solvant organique dans lequel les esters de formule I ne sont pas ou seulement lentement solvolysables, ou par voie chromatographique.

9. Procédé suivant la revendication 8, caractérisé en ce que l'on effectue la séparation par chromatographie par adsorption sur des colonnes de gel de silice avec un mélange d'acétone/isopropanol en tant qu'éluant.

10. Procédé suivant la revendication 8, caractérisé en ce que l'on effectue la séparation par chromatographie liquide sous haute pression avec un système à phases inversées.

11. Procédé suivant la revendication 10, caractérisé en ce qu'un mélange de méthanol/tampon sert de phase mobile.

12. Application des monoesters du céliprolol suivant les revendications 1 à 11 à la préparation de formes du céliprolol optiquement pures par clivage hydrolytique.

13. Application suivant la revendication 12, caractérisée en ce que les esters optiquement purs suivant la revendication 8 sont soumis à la séparation.